# EUROPEAN PATENT SPECIFICATION

(11) **EP 1 888 040 B1**
(45) Date of publication and mention of the grant of the patent: **18.08.2010**
(21) Application number: 06759225.3
(22) Date of filing: 08.05.2006
(51) Int. Cl.: A61K 9/20, A61K 9/16, A61K 31/506

(54) **PHARMACEUTICAL COMPOSITIONS COMPRISING IMATINIB AND A RELEASE RETARDANT**
PHARMAZEUTISCHE ZUSAMMENSETZUNGEN MIT IMATINIB UND EINEM FREISETZUNGSVERZÖGERER
COMPOSITIONS PHARMACEUTIQUES COMPRENANT L'IMATINIBE ET UN RETARDATEUR DE LIBERATION

(30) Priority: 10.05.2005 US 679607 P
(43) Date of publication of application: 20.02.2008
(73) Proprietor: Novartis AG, 4056 Basel (CH); Novartis Pharma GmbH, 1230 Wien (AT)
(72) Inventor: VASANTHAVADA, Madhav, Basking Ridge, New Jersey 07920 (US); LAKSHMAN, Jay Parthiban, Cedar Knolls, New Jersey 07927 (US); TONG, Wei-Qin, Basking Ridge, New Jersey 07920 (US); SERAJUDDIN, Abu T.M., Flushing, New York 11358 (US)
(74) Representative: Roth, Peter Richard
(86) International application number: PCT/US2006/017558
(87) International publication number: WO 2006/121941

(56) References cited:
- WO-A-03/077892
- WO-A-03/090720
- WO-A-03/094904
- WO-A-2006/040779
- WO-A-2006/048890
- WO-A1-2006/082099

## Description

### Field of the Invention

The present invention relates to sustained release pharmaceutical compositions comprising imatinib, and a release retardant. The present invention also relates to processes for making such sustained release pharmaceutical compositions.

### Background of the Invention

The therapeutic compound 4-[(4-methyl-1-piperazinyl)methyl]-*N*-[4-methyl-3-[[4-(3-pyridinyl)-2-pyrimidinyl]amino]phenyl]-benzamide, or more commonly known as imatinib, and its preparation are described in U.S. Patent No. 5,521,184.

Basic pharmaceutically active therapeutic compounds are commonly formulated into pharmaceutical preparations as an acid addition salt form, particularly as a crystalline acid addition salt. For example, imatinib is marketed In many countries as its monomethanesulfonate salt (imatinib mesylate) under the brandname GLIVEC or GLEEVEC. Two crystal forms of imatinib mesylate are described in WO 99/03854. The crystal form designated as the beta form is described as having physical properties that make it advantageous for the manufacture of solid oral pharmaceutical dosage forms, such as tablet and capsule dosage forms.

The currently marketed formulations of imatinib mesylate are 100 mg hard gelatin capsules and 100 mg and 400 mg film coated tablets. WO 03/090720 discloses tablets comprising imatinib mesylate which are prepared by means of wet granulation. There is a need for an extended release tablet comprising imatinib, for example, to reduce peak plasma concentration and to maintain therapeutic plasma levels for a prolonged period of time.

It is an object of the present invention to provide for a sustained release formulation for imatinib. It is a further object of the present invention to provide for a sustained release formulation manufactured by using a melt granulation process. It is yet another object of the present invention to provide for the use of an extruder to implement the melt granulation process. Traditionally, extruders in a pharmaceutical context have been used for the manufacture of solid dispersion and/or solid solutions that have required at least a partial melting of the therapeutic compound. Surprisingly, it has been found that the use of extruders can be useful in the preparation of melt granulated solid dosage forms without the need for melting imatinib or a pharmaceutically acceptable salt thereof.

### Summary of the Invention

The present invention relates to the modified release pharmaceutical compositions of claim 1 that contain imatinib or a pharmaceutically acceptable salt thereof and a release retardant. The amount of imatinib in the pharmaceutical composition can be at least 50% by weight of the composition. The balance of the pharmaceutical composition can be made up of at least one release retardant. In a particular aspect of the present invention the release retardant is a water-soluble, water swellable and/or water insoluble polymer. Particularly useful as such polymers are ethylcellulose, hydroxypropyl cellulose and/or hydroxypropyl methyl cellulose. in yet another aspect the release retardant can be a non-polymeric release retardant. In a particular aspect, the non-polymeric release retardant is hydrogenated castor oil. The aforementioned compositions can be milled or granulated and compressed into monolithic tablets or encapsulated into capsules.

In another exemplary embodiment of the present invention, the invention features the method of claim 13 for making sustained release pharmaceutical compositions of imatinib or a pharmaceutically acceptable salt thereof. In a particular aspect, imatinib or a pharmaceutically acceptable salt thereof is melt granulated with a release retardant using an extruder. During the processing in the extruder, the heating temperature of the extruder does not exceed the melting temperature of imatinib or a pharmaceutically acceptable salt thereof. The result extrudate can be optionally milled and compressed into solid oral dosage forms.

### Brief Description of the Drawings

The accompanying drawings, which are incorporated in and constituting a part of the specification, illustrates exemplary embodiments of the present invention.
FIG. 1 shows a chart depicting the dissolution profiles for exemplary embodiments in accordance with the present invention as disclosed in Examples 1, 2 and 3.
FIG. 2 shows a chart depicting the dissolution profiles for present invention as disclosed in Examples 4, 5, 6 and 7.

### Detailed Description of the Invention

The present invention relates to sustained release solid dosage forms of imatinib or a pharmaceutically acceptable salt thereof which comprises granules of the therapeutic compound with a release retardant and to a process for preparing such dosage forms. The sustained release solid dosage forms may optionally further comprise plasticizers, release modifier, disintegrants, and/or lubricants.

As used herein the term "pharmaceutical composition" means a mixture (e.g., a solid dispersion) and/ or solution (e.g., a solid solution) containing a therapeutic compound to be administered to a mammal, e.g., a human in order to prevent, treat or control a particular disease or condition affecting the mammal. The term "pharmaceutical composition" as used herein, for example, also encompasses an intimate physical mixture formed at high temperature and pressure.

As used herein the term "pharmaceutically acceptable" refers to those compounds, materials, compositions and/or dosage forms, which are, within the scope of sound medical judgment, suitable for contact with the tissues of mammals, especially humans, without excessive toxicity, irritation, allergic response and other problem complications commensurate with a reasonable benefit/risk ratio.

As used herein the term "therapeutic compound" means any compound, substance, drug, medicament, or active ingredient having a therapeutic or pharmacological effect, and which is suitable for administration to a mammal, e.g., a human, in a composition that is particularly suitable for oral administration. Particularly useful as a therapeutic compound in the present invention is imatinib and pharmaceutically acceptable salts thereof.

As used herein the term "imatinib" refers to the free base of imatinib or a pharmaceutically acceptable salt thereof (e.g., imatinib mesylate).

Pharmaceutically acceptable salts of imatinib include, but are not limited to, pharmaceutically acceptable acid addition salts. Examples include inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic arid, heteroaromatic carboxylic acids, such as nicotinic acid or isonicotinic acid, aliphatic sulfonic acids, such as methane-, ethane- or 2-hydroxyethane-sulfonic acid, or aromatic sulfonic acids, for example benzene-, p-toluene- or naphthalene-2-sulfonic acid. Other examples of acid addition salts include tartrate salt, such as a (*D*)(-) tartrate salt or a (*L*)(+) tartrate salt, a hydrochloride salt, a citrate salt, a malate salt, particularly a *D*-malate salt, a fumarate salt, a succinate salt, a benzoate salt, a benzenesulfonate salt, a pamoate salt, a formate salt, a malonate salt, a 1,5-naphthalenedisulfonate salt, a salicylate salt, a cyclohexanesulfamate salt, a lactate salt, particularly a (*S*)-lactate salt, a mandelate salt, particularly an (*R*)(-) mandelate salt, a glutarate salt, an adipate salt, a squarate salt, a vanillate salt, an oxaloacetate salt, an ascorbate salt, particularly an (*L*)-ascorbate salt and a sulfate salt.

In one exemplary embodiment, the acid addition salt is selected from the group consisting of imatinib ascorbate, imatinib formate, imatinib malonate, imatinib oxaloacetate, imatinib squarate and imatinib vanillate.

The monomethanesulfonic acid addition salt of imatinib and an exemplary crystal form thereof, e.g. the beta-crystal form, are described in PCT patent application WO99/03854 published on January 28, 1999. Imatinib mesylate has an aqueous solubility of >1,300 mg/mL at pH of less than 5.5.

The therapeutic compound(s) is present in the pharmaceutical compositions of the present invention in a therapeutically effective amount or concentration. Such a therapeutically effective amount or concentration is known to one of ordinary skill in the art as the amount or concentration varies with the therapeutic compound being used and the indication which is being addressed. In accordance with the present invention, the therapeutic compound imatinib may be present in an amount of 50% to 99% by weight of pharmaceutical composition. In one embodiment, imatinib, may be present in an amount by weight of 62% to 99% by weight of the pharmaceutical composition. In one embodiment, imatinib may be present in an amount by weight of 75% to 99% by weight of the pharmaceutical composition.

As used herein, the term "immediate release" refers to the rapid release of the majority of the therapeutic compound, e.g., greater than 50%, 60%, 70%, 80%, or 90% within a relatively short time, *e*.*g*., within 1 hour, 40 minutes, 30 minutes or 20 minutes after oral ingestion. Particularly useful conditions for immediate release are release of at least or equal to 80% of the therapeutic compound within thirty minutes after oral ingestion. The particular immediate release conditions for a specific therapeutic compound will be recognized or known by one of ordinary skill in the art.

As used herein, the term "sustained release", or modified release, refers to the gradual but continuous or sustained release over a relatively extended period of the therapeutic compound content after oral ingestion. The release will continue over a period of time and may continue through until and after the pharmaceutical composition reaches the intestine. Sustained release may also refer to delayed release in which release of the therapeutic compound does not start immediately when the pharmaceutical composition reaches the stomach but is delayed for a period of time, for instance, until when the pharmaceutical composition reaches the intestine when the increasing pH is used to trigger release of the therapeutic compound from the pharmaceutical composition.

As used herein the term "release retardant" refers to any material or substance that slows the release of a therapeutic compound from a pharmaceutical composition when orally ingested. Various sustained release systems, as known in the art, can be accomplished by the use of a release retardant, e.g., a diffusion system, a dissolution system and/or an osmotic system. A release retardant can be a polymer or non-polymer.

As used herein the term "polymer" refers to a polymer or mixture of polymers that has a glass transition temperature, softening temperature or melting temperature less than 212°C. The glass transition temperature is the temperature at which such polymer's characteristics change from that of highly viscous to that of relatively less viscous mass. Types of polymers include, but are not limited to, water-soluble, water-swellable, water insoluble polymers and combinations of the foregoing.

Examples of polymers include, but are not limited to:
homopolymers and copolymers of N-vinyl lactams, *e*.*g*., homopolymers and copolymers of N-vinyl pyrrolidone (e.g., polyvinylpyrrolidone), copolymers of N-vinyl pyrrolidone and vinyl acetate or vinyl propionate;
cellulose esters and cellulose ethers (e.g., methylcellulose and ethylcellulose) hydroxyalkylcelluloses (e.g., hydroxypropylcellulose), hydroxyalkylalkylcelluloses (e.g., hydroxypropylmethylcellulose), cellulose phthalates (e.g., cellulose acetate phthalate and hydroxylpropylmethylcellulose phthalate) and cellulose succinates (e.g., hydroxypropylmethylcellulose succinate or hydroxypropylmethylcellulose acetate succinate);
high molecular polyalkylene oxides such as polyethylene oxide and polypropylene oxide and copolymers of ethylene oxide and propylene oxide;
polyacrylates and polymethacrylates (e.g., methacrylic acid/ethyl acrylate copolymers, methacrylic acid/methyl methacrylate copolymers, butyl methacrylate/2-dimethylaminoethyl methacrylate copolymers, poly(hydroXyalkyl acrylates), poly(hydroxyalkyl methacrylates));
polyacrylamides;
vinyl acetate polymers such as copolymers of vinyl acetate and crotonic acid, partially hydrolyzed polyvinyl acetate;
polyvinyl alcohol; and
oligo- and polysaccharides such as carrageenans, galactomannans and xanthan gum, or mixtures of one or more thereof.

As used herein, the term "plasticizer" refers to a material that may be incorporated into the pharmaceutical composition, especially the internal phase, in order to decrease the glass transition temperature and the melt viscosity of a polymer by increasing the free volume between polymer chains. Plasticizers, for example, include, but are not limited to, water; citrate esters (e.g., triethylcitrate, triacetin); low molecular weight poly(alkylene oxides) (e.g., poly(ethylene glycols), poly(propylene glycols), poly(ethylene/propylene glycols)); glycerol, pentaerythritol, glycerol monoacetate, diacetate or triacetate; propylene glycol; sodium diethyl sulfosuccinate; and the therapeutic compound itself. The plasticizer can be present in concentration from 0% to 15%, e.g., 0.5% to 5% by weight of the pharmaceutical composition. Examples of plasticizers can also be found in The Handbook of Pharmaceutical Additives, Ash et al., Gower Publishing (2000).

As used herein the term "non-polymeric release retardant" refers to substances or a mixtures of substances, non-polymeric in nature, that are solid or semi-solid at room temperature (25°C) and with melting points (or melting ranges) less than or approximately equal to the melting range of imatinib or a pharmaceutically acceptable salt thereof.

Particularly useful as non-polymeric release retardants are hydrophobic non-polymeric release retardants. As used herein, the term "hydrophobic", with respect to the release retardant, refers to being more compatible with oil than with water. A substance with hydrophobic properties is insoluble or almost insoluble in water but is easily soluble in oil or other nonpolar solvents.

Examples of hydrophobic non-polymeric release retardants include, but are not limited to, esters, hydrogenated oils, natural waxes, synthetic waxes, hydrocarbons, fatty alcohols, fatty acids, monoglycerides, diglycerides, triglycerides and mixtures thereof.

Examples of esters, such as glyceryl esters include, but are not limited to, glyceryl monostearate, e.g., CAPMUL GMS from Abitec Corp. (Columbus, OH); glyceryl palmitostearate; acetylated glycerol monostearate; sorbitan monostearate, e.g., ARLACEL 60 from Uniqema (New Castle, DE); and cetyl palmitate, e.g., CUTINA CP from Cognis Corp. (DOsseldort, Germany), magnesium stearate and calcium stearate.

Examples of hydrogenated oils include, but are not limited to, hydrogenated castor oil; hydrogenated cottonseed oil; hydrogenated soybean oil; and hydrogenated palm oil. An example of oil includes sesame oil.

Examples of waxes include, but are not limited to, carnauba wax, beeswax and spermaceti wax. Examples of hydrocarbons include, but are not limited to, microcrystalline wax and paraffin. Examples of fatty alcohols, i.e., higher molecular weight nonvolatile alcohols that have from 14 to 31 carbon atoms include, but are not limited to, cetyl alcohol, e.g., CRODACOL C-70 from Croda Corp. (Edison, NJ) ; stearyl alcohol, e.g., CRODACOL S-95 from Croda Corp; lauryl alcohol; and myristyl alcohol. Examples of fatty acids which may have from 10 to 22 carbon atoms include, but are not limited to, stearic acid, e.g., HYSTRENE 5016 from Crompton Corp. (Middlebury, CT); decanoic acid; palmitic acid; lauric acid; and myristic acid.

As used herein the term "release modifier" refers to substance or mixture of substances that would help to provide either enhancement or retardation in release profile as a function of pH. Release modifier could be polymeric or non-polymerc in nature, solid or semi-solid at room temperature (25°C), and with melting points less than or approximately equal to melting range of imatinib or a pharmaceutically acceptable salt thereof. A release modifier, for example, would help in enhanced drug release at higher pH conditions, where the solubility of imatinib or its salt is lower than that in acid condition.

Examples of polymeric release modifiers include, but are not limited to, water soluble polymers that exhibit charge in their dissolved state, as a function of pH. Examples are methacrylate polymers, polymers containing quaternary ammonium or acetate groups.

Particularly useful non-polymerc release modifiers include, but are not limited to, water soluble surface active agents. More specifically, the surfactants could exhibit charge in their dissolved state, as a function of pH. Examples of such surfactants are sodium lauryl sulfate, and block-co-polymers with ionizable groups.

As used herein, the term "melt granulation" refers to the following compounding process that comprises the steps of:
(a) forming a mixture of a therapeutic compound with at least one release retardant, e.g. a release retarding polymer, and optionally, a plasticizer or a release modifier;
(b) granulating the mixture using an extruder or other suitable equipment, for example a jacketed high shear mixer, while heating the mixture to a temperature above the softening temperature of the release retardant; as used herein, the "softening temperature" refers to the temperature at which the release retardant experiences a change in the rate of viscosity decrease as a function of temperature; and
(c) cooling the granules to a temperature less than 50°C, for example to room temperature (25°C).
As described in this embodiment, the term granules shall be used interchangeably with melt granules. "Melt granules" as used herein also refers to any form of solid or semi-solid mass exiting the extruder. For example, melt granules could be noodle-shaped mass, powder or aggregation of powder.

The heating and mixing of the therapeutic compound and the release retardant to form an internal phase of granules may be accomplished, e.g., by the use of a fluidized bed granulator, an extruder or a vessel supplied with high-shear mixing means. The release retardant, e.g., can be present in an amount from 1% to 50% by weight of the composition In one embodiment, the release retardant may be present in an amount from 3 to 25% by weight of the composition. The therapeutic compound may be present in an amount from 50% to 99% by weight of the composition. In one embodiment, the therapeutic compound may be present in an amount of 60% to 97%.

The resulting granules are, for example, particles of the therapeutic compound coated or substantially coated by the release retardant, or alternatively, particles of the therapeutic compound embedded or substantially embedded with or within the release retardant.

Particularly useful for effecting the melt granulation process is an extruder. In general, an extruder includes a rotating screw(s) within a stationary barrel with a die located at one end of the barrel. Along the entire length of the screw, distributive mixing of the materials (e.g., the therapeutic compound, release retarding material, and any other needed excipients) is provided by the rotation of the screw(s) within the barrel. Conceptually, the extruder can be divided into at least three sections: a feeding section; a heating section and a metering section. In the feeding section, the raw materials are fed into the extruder, e.g. from a hopper. The raw materials can be directly added to the hopper without the need of a solvent. In the heating section, the raw materials are heated to a temperature greater than the softening temperature of the release retarding material. After the heating section is a metering section in which the mixed materials are extruded through a die into a particular shape, e.g., granules or noodles. Types of extruders particularly useful in the present invention are single-, twin- and multi-screw extruders, optionally configured with kneading paddles.

Once the granules are obtained, the granules may be formulated into oral forms, e.g., solid oral dosage forms, such as tablets, pills, lozenges, caplets, capsules or sachets, by adding additional conventional excipients which comprise an external phase of the pharmaceutical composition. Examples of such excipients include, but are not limited to, disintegrants, plasticizers, binders, lubricants, glidants, stabilizers, fillers and diluents. One of ordinary skill in the art may select one or more of the aforementioned excipients with respect to the particular desired properties of the solid oral dosage form by routine experimentation and without any undue burden. The amount of each excipient used may vary within ranges conventional in the art. The following references disclose techniques and excipients used to formulate oral dosage forms. See The Handbook of Pharmaceutical Excipients, 4th edition, Rowe et al., Eds., American Pharmaceuticals Association (2003); and Remington: the Science and Practice of Pharmacy, 20th edition, Gennaro, Ed., Lippincott Williams & Wilkins (2003).

Examples of pharmaceutically acceptable disintegrants include, but are not limited to starches; clays; celluloses; alginates; gums; cross-linked polymers, e.g., cross-linked polyvinyl pyrrolidone or crospovidone, e.g., POLYPLASDONE XL from International Specialty Products (Wayne, NJ); cross-linked sodium carboxymethylcellulose or croscarmellose sodium, e.g., AC-DI-SOL from FMC; and cross-linked calcium carboxymethylcellulose; soy polysaccharides; and guar gum. The disintegrant may be present in an amount from 0% to 10% by weight of the composition. In one embodiment, the disintegrant is present in an amount from 0.1 % to 1.5% by weight of composition.

Examples of pharmaceutically acceptable binders include, but are not limited to, starches; celluloses and derivatives thereof, for example, microcrystalline cellulose, e.g., AVICEL PH from FMC (Philadelphia, PA), hydroxypropyl cellulose hydroxylethyl cellulose and hydroxylpropylmethyl cellulose METHOCEL from Dow Chemical Corp. (Midland, MI); sucrose; dextrose; corn syrup; polysaccharides; and gelatin. The binder may be present in an amount from 0% to 50%, e.g., 10-40% by weight of the composition.

Examples of pharmaceutically acceptable lubricants and pharmaceutically acceptable glidants include, but are not limited to, colloidal silica, magnesium trisilicate, starches, talc, tribasic calcium phosphate, magnesium stearate, aluminum stearate, calcium stearate, magnesium carbonate, magnesium oxide, polyethylene glycol, powdered cellulose and microcrystalline cellulose. The lubricant may be present in an amount from 0% to 10% by weight of the composition. In one embodiment, the lubricant may be present in an amount from 0.1% to 1.5% by weight of composition. The glidant may be present in an amount from 0.1% to 10% by weight.

Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc. The filler and/or diluent, e.g., may be present in an amount from 15% to 40% by weight of the composition.

To make pharmaceutical compositions of the present invention, imatinib or a pharmaceutically acceptable salt thereof and a release retardant are blended in a ratio in a range of 99:1 to 1:1 (on a dry weight basis) prior to, or upon addition into the hopper of an extruder. Optionally, a plasticizer is added to the internal phase.

The mixture is heated to a temperature above the softening temperature, melting temperature, or glass transition temperature of the release retardant; however, the heating temperature does not exceed the melting temperature of the therapeutic compound. As the mixture is being heated, it is also being kneaded by the screw(s) of the extruder. The mixture is maintained at the elevated temperature and blended for a time sufficient to form a granulated product. After the mixture is conveyed down the entire length of the barrel, a granulated product is obtained, the granulated mixture is cooled.

After cooling, the granules may be milled and subsequently screened through a sieve. The granules (which constitute the internal phase of the pharmaceutical composition) are then combined with solid oral dosage form excipients (the external phase of the pharmaceutical composition), i.e., fillers, binders, disintegrants and lubricants. The combined mixture may be further blended, e.g., through a V-blender, and subsequently compressed or molded into a tablet, for example a monolithic tablet, or encapsulated by a capsule.

Once the tablets are obtained, they can be optionally coated with a functional or non-functional coating as known in the art. Examples of coating techniques include, but are not limited to, sugar coating, film coating, microencapsulation and compression coating. Types of coatings include, but are not limited to, enteric coatings, sustained release coatings, controlled-release coatings.

The utility of all the pharmaceutical compositions of the present invention may be observed in standard clinical tests in, for example, known indications of drug dosages giving therapeutically effective blood levels of the therapeutic compound; for example using dosages in the range of 2.5-1000 mg of therapeutic compound per day for a 75 kg mammal, e.g., adult and in standard animal models.

The pharmaceutical composition, e.g., in form of a tablet or a powder suitable for tablet formulation will suitably contain at least 400 mg of imatinib or a pharmaceutically acceptable salt thereof. In one embodiment, the tablet formulation will contain 800mg of imatinib or a pharmaceutically acceptable salt thereof.
Such unit dosage forms are suitable for administration one to two times daily depending upon the particular purpose of therapy, the phase of therapy and the like.

The present invention provides a method of treatment of a subject suffering from a disease, condition or disorder treatable with a therapeutic compound comprising administering a therapeutically effective amount of a pharmaceutical composition of the present invention to a subject in need of such treatment.

Additionally, the present invention provides the use of a composition according to the present invention comprising imatinib mesylate in the manufacture of a medicament for the treatment and/or prevention of conditions, such as malignant or non-malignant proliferative disorders; inhibition of angiogenesis; leukemias such as chronic myelomonocytic leukemia, chronic myeloid leukemia or acute lymphocytic leukemia, gliomas, glioblastoma multiforme, sarcomas; tumors of prostate, colon, breast, lung, or ovary, atherosclerosis, thrombosis; sclerodermitis; psoriasis, restenosis, fibrosis; asthma, prevention of transplantation induced disorders, e.g. obliterative bronchiolitis; prevention of cell invasion by certain bacteria, like Porphyromonas gingivalis; of multi-drug resistance, hypereosinophilic syndrome, gastrointestinal stromal tumors (GIST), dermatofibrosarcoma protuberans, systemic mastocytosis or, more generally, Philadelphia positive myeloproliferative disorders.

In summary, the present invention relates, but is not limited, to the following aspects:
(A) A pharmaceutical composition comprising imatinib or an of its salts and a release retardant, especially comprising between 50% and 99% by weight Imatinib, more particular between 62% and 99% by weight of Imatinib;
   - In particular said pharmaceutical composition, wherein said composition comprises at least 400 mg of imatinib mesylate;
   - In particular said pharmaceutical composition, wherein said release retardant is a polymer, especially wherein said polymer has a glass transition temperature less than the melting point of imatinib mesylate, optionally further comprises a plasticizer;
   - In particular said pharmaceutical composition, wherein said release retardant is a non-polymeric release retardant, in particular wherein said non-polymeric release retardant melts at a temperature less than the melting point of imatinib or the imatinib salt employed and/or wherein said non-polymeric release retardant is hydrogenated castor oil;
   - In particular said pharmaceutical composition, wherein the pharmaceutical composition comprises at least one release retardant selected from the group consisting of water soluble, water insoluble and water swellable cellulose polymers, acrylic polymers, polysaccharides and polyols;
   - In particular said pharmaceutical composition, wherein the pharmaceutical composition comprises at least one release retardant selected from the group consisting of hydroxypropyl cellulose, hydroxypropylmethyl cellulose, ethylcellulose and methacrylate polymers;
   - In particular said, pharmaceutical composition, further comprising a release modifier.
   - In particular said pharmaceutical composition, where the drug release from the pharmaceutical composition is not greater than 80% at 1 hour, and not less than 80% at 10 hours, when tested using USP I basket apparatus at 50rpm in 900mL of 0.1 N hydrochloric acid at 37°C.
   - In particular said pharmaceutical composition, where the drug release from the pharmaceutical composition is not greater than 80% at 2 hours and not less than 80% at 8 hours, when tested using USP I basket apparatus at 50rpm in 900mL of 0.1 N hydrochloric acid at 37°C.
   - In particular said pharmaceutical composition, wherein the composition provides, in healthy humans, a mean plasma concentration value not exceeding 3.5µg Imatinib/mL, when dosed 2 hours after a light breakfast.
(B) A method of making a modified release pharmaceutical composition comprising the step of granulating imatinib or any of its salts with a release retardant and optionally a release modifier in an extruder while heating to a temperature below the melting temperature of imatinib or its salt, to form melt granules;
   - In particular said method, wherein the melt granules are once again introduced into the extruder, to further granulate with or without release retardant or release modifier or plasticizer, at a temperature less than the melting point of imatinib or the imatinib salt employed;
   - In particular said method, further comprising cooling of said melt granules to a desired temperature, which is less than melt granulation process temperature.
   - In particular said method, further comprising compressing the melt granules into a tablet, e.g. wherein melt granules manufactured separately using different release retardants and/or release modifiers and/or plasticizers at different compositions are blended and compressed into a tablet.
   - In particular said method, wherein said extruder is a twin-screw extruder, and, more especially, wherein the release retardant is a polymer, more specifically hydroxypropyl cellulose;
   - In particular said method, wherein said composition comprises at least 50% imatinib by weight of the composition.

The following examples are illustrative, but do not serve to limit the scope of the invention described herein. The examples are meant only to suggest a method of practicing the present invention.

Quantities of ingredients, represented by percentage by weight of the pharmaceutical composition, used in each example are set forth below.

### Example 1

| **Ingredient** | **Percentage (w/w)** | **Amount per tablet (mg)** |
|---|---|---|
| Internal phase | | |
| imatinib mesylate | 89% | 956 |
| hydroxypropyl methylcellulose | 5% | 53 |
| hydroxypropyl cellulose | 5% | 53 |
| External phase | | |
| magnesium stearate | 1% | 10 |
| Total | | 1072 |

The internal phase ingredients: imatinib mesylate, hydroxypropyl methylcellulose available as METHOCEL K 100M Premium CR from Dow Chemical Co. (Midland, Michigan), hydroxypropyl cellulose available as KLUCEL HF Pharm from Hercules Chemical Co. (Wilmington, Delaware) are combined and blended in a bin blender for about two hundred rotations. Subsequent to blending, the internal phase is introduced into the feed section, or hopper, of a twin screw extruder. A suitable twin screw extruder is the PRISM 16 mm pharmaceutical twin screw extruder available from Thermo Electron Corp. (Waltham, Massachusetts).

Located at the end of the twin screw extruder is a die with a bore of approximately three mm. The twin screw extruder is configured with five individual barrel zones, or sections. Starting from the hopper to the die, the zones are respectively heated to the following temperatures: 40°C, 70°C, 110°C, 150°C and 185°C.

As the material progresses through the extruder, the speed of the screws is gradually increased to 150 rpm and the volumetric feed rate is adjusted to deliver between about twelve to fifteen grams of material per minute.

The extrudate, or granules, from the extruder are then cooled to room temperature by allowing them to stand from approximately fifteen to twenty minutes. The cooled granules, are subsequently sieved through an 18 mesh screen (i.e., a one mm screen).

For the external phase, the magnesium stearate is first passed through an 18 mesh screen. The magnesium stearate is then blended with the obtained granules from the internal blender in a bin blender for approximately sixty rotations. The resulting final blend is compressed into tablets using a conventional rotary tablet press (e.g., Manesty Beta Press). The resulting tablets are monolithic and have a hardness in the range of 15 kP to 33 kP.

### Example 2

| **Ingredient** | **Percentage (w/w)** | **Amount per tablet (mg)** |
|---|---|---|
| Internal phase | | |
| imatinib mesylate | 94% | 956 |
| hydroxypropyl cellulose | 5% | 50 |

| External phase | | |
|---|---|---|
| magnesium stearate | 1% | 10 |
| Total | | 1016 |

The tablets of Example 2 are made using the same method as disclosed for Example 1; however, no hydroxypropyl methylcellulose is added to the internal phase.

### Example 3

| **Ingredient** | **Percentage (w/w)** | **Amount per tablet (mg)** |
|---|---|---|
| Internal phase | | |
| imatinib mesylate | 94% | 956 |
| hydroxypropyl methylcellulose | 4% | 40 |
| hydrogenated castor oil | 1 % | 10 |

| External phase | | |
|---|---|---|
| magnesium stearate | 1% | 10 |
| Total | | 1016 |

The tablets of Example 3 are made using the same method as disclosed in Example 1; however, hydrogenated castor oil available as CUTINA HR from Cognis Corp. (Düsseldorf, Germany) is added to the internal phase.

### Example 4

| **Ingredient** | **Percentage (w/w)** | **Amount per tablet (mg)** |
|---|---|---|
| Internal phase | | |
| imatinib mesylate | 89% | 956 |
| hydroxypropyl methylcellulose | 5% | 53 |
| ethylcellulose | 5% | 53 |

| External phase | | |
|---|---|---|
| magnesium stearate | 1% | 10 |
| Total | | 1072 |

The tablets of Example 4 are manufactured using the method described in Example 1. In the present, hydroxypropyl cellulose is replaced by ethyl cellulose.

### Example 5

| **Ingredient** | **Percentage (w/w)** | **Amount per tablet (mg)** |
|---|---|---|
| Internal phase | | |
| imatinib mesylate | 89% | 956 |
| ethylcellulose | 10% | 106 |

| External phase | | |
|---|---|---|
| magnesium stearate | 1% | 10 |
| Total | | 1072 |

The tablets of Example 5 are manufactured using the method described in example 4, however, hydroxypropylmethylcellulose is replaced by ethylcellulose.

### Example 6

| **Ingredient** | **Percentage (w/w)** | **Amount per tablet (mg)** |
|---|---|---|
| Internal phase | | |
| imatinib mesylate | 84% | 956 |
| ethylcellulose | 10% | 168 |
| Sodium lauryl sulfate | 5% | 84 |

| External phase | | |
|---|---|---|
| magnesium stearate | 1% | 12 |
| Total | | 1221 |

The tablets of Example 6 are manufactured using the method described in eExample 1. However, a release modifier - sodium lauryl sulfate, is incorporated in the internal phase.

### Example 7

### Melt granules A

| **Ingredient** | **Percentage (w/w)** | **Amount per tablet (mg)** |
|---|---|---|
| Internal phase | | |
| imatinib mesylate | 89% | 956 |
| Hydroxypropylcellulose | 10% | 106 |

| External phase | | |
|---|---|---|
| magnesium stearate | 1% | 10 |
| Total | | 1072 |

### Melt granules B

| **Ingredient** | **Percentage (w/w)** | **Amount per tablet (mg)** |
|---|---|---|
| Internal phase | | |
| imatinib mesylate | 89% | 956 |
| ethylcellulose | 10% | 106 |

| External phase | | |
|---|---|---|
| magnesium stearate | 1% | 10 |
| Total | | 1072 |

Melt granules A and melt granules B are manufactured separately and are combined prior to compression. The ratio of melt granules A to melt granules B is 85:15.

### Example 8

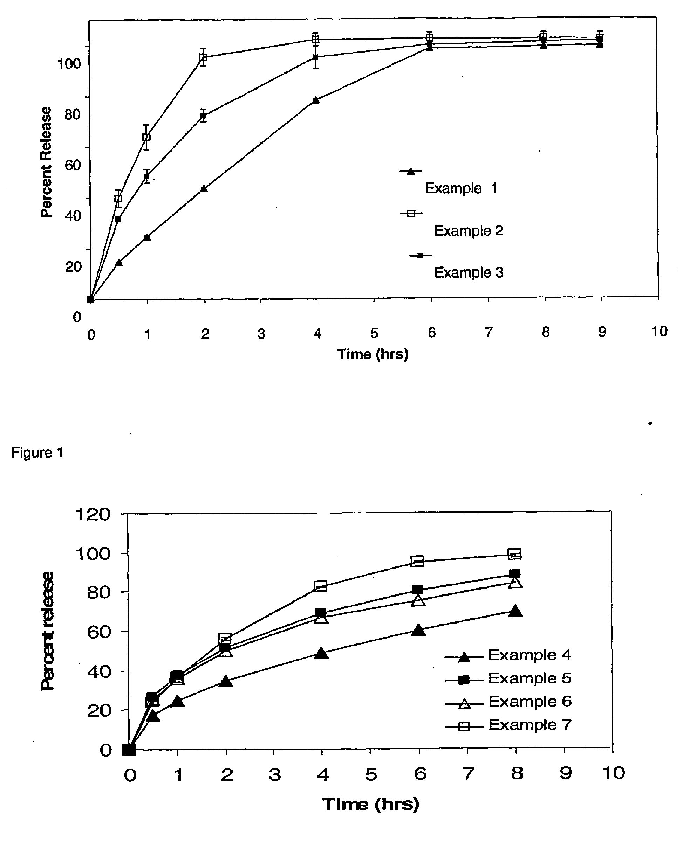

FIG. 1 is a chart showing the dissolution profile for tablets for each of the three Examples 1-3. The tablets are placed in 900 mL of 0.1 N HCl (pH 1.2) using USP Apparatus II rotating at 100 rpm and at 37°C. The in vitro release profiles of the compositions described in Examples 4, 5, 6 and 7 is shown in FIG 2. The charts show that the Examples of the present invention have a sustained release profile.

## Claims

1. An oral sustained release pharmaceutical composition comprising melt granules of imatinib or any of its salts and a release retardant, and which comprises at least 400 mg of imatinib mesylate.

2. The pharmaceutical composition of Claim 1, comprising between 50% and 99 % by weight of imatinib.

3. The pharmaceutical composition of Claim 2, wherein said composition comprises between 62% and 99 % by weight of imatinib.

4. The pharmaceutical composition of Claim 1, wherein said release retardant is a polymer.

5. The pharmaceutical composition of Claim 4, wherein said polymer has a glass transition temperature less than the melting point of imatinib mesylate.

6. The pharmaceutical composition of Claim 5, further comprising a plasticizer.

7. The pharmaceutical composition of Claim 1, wherein said release retardant is a non-polymeric release retardant.

8. The pharmaceutical composition of Claim 7, wherein said non-polymeric release retardant melts at a temperature less than the melting point of imatinib or the imatinib salt employed.

9. The pharmaceutical composition of Claim 7, wherein said non-polymeric release retardant is hydrogenated castor oil.

10. The pharmaceutical composition of Claim 1, wherein the pharmaceutical composition comprises at least one release retardant selected from the group consisting of water soluble, water insoluble and water swellable cellulose polymers, acrylic polymers, polysaccharides and polyols.

11. The pharmaceutical composition of Claim 1, wherein the pharmaceutical composition comprises at least one release retardant selected from the group consisting of hydroxypropyl cellulose, hydroxypropylmethyl cellulose, ethylcellulose and methacrylate polymers.

12. The pharmaceutical composition according to any one of Claims 1 to 11, further comprising a release modifier.

13. A method of making an oral sustained release pharmaceutical composition comprising the step of melt granulating imatinib or any of its salts with a release retardant and optionally a release modifier in an extruder while heating to a temperature below the melting temperature of imatinib or its salt, to form melt granules, and wherein the composition comprises at least 50% imatinib by weight of the composition.

14. The method of claim 13, wherein the melt granules are once again Introduced into the extruder, to further granulate with or without release retardant or release modifier or plasticizer, at a temperature less than the melting point of imatinib or the imatinib salt employed.

15. The method of claim 13, further comprising cooling of said melt granules to a desired temperature, which is less than melt granulation process temperature.

16. The method of Claim 13, further comprising compressing the melt granules into a tablet.

17. The method of Claim 16, wherein melt granules manufactured separately using different release retardants and/or release modifiers and/or plasticizers at different compositions are blended and compressed into a tablet.

18. The method of Claim 13, wherein said extruder is a twin-screw extruder.

19. The method of Claim 14, wherein the release retardant is a polymer.

20. The method of Claim 19, wherein said polymer is hydroxypropyl cellulose.

21. The pharmaceutical composition according to any one of claims 1 to 12, where the drug release from the pharmaceutical composition is not greater than 80% at 1 hour, and not less than 80% at 10 hours, when tested using USP I basket apparatus at 50rpm in 900mL of 0.1 N hydrochloric acid at 37°C.

22. The pharmaceutical composition according to any one of claims 1 to 12, where the drug release from the pharmaceutical composition is not greater than 80% at 2 hours and not less than 80% at 8 hours, when tested using USP I basket apparatus at 50rpm in 900mL of 0.1N hydrochloric acid at 37°C.

23. The pharmaceutical composition according to any one of claims 1 to 12, wherein the composition provides, in healthy humans, a mean plasma concentration value not exceeding 3.5µg Imatinib/mL, when dosed 2 hours after a light breakfast.

## Patentansprüche

1. Orale, für eine verlängerte Freisetzung sorgende pharmazeutische Zusammensetzung, die ein Schmelzgranulat von Imatinib oder einem beliebigen der Salze hiervon und ein Freisetzungsverzögerungsmittel umfasst, und die mindestens 400 mg Imatinibmesylat umfasst.

2. Pharmazeutische Zusammensetzung nach Anspruch 1, die zwischen 50 und 99 Gew.-% Imatinib umfasst.

3. Pharmazeutische Zusammensetzung nach Anspruch 2, wobei die Zusammensetzung zwischen 62 und 99 Gew.-% Imatinib umfasst.

4. Pharmazeutische Zusammensetzung nach Anspruch 1, wobei das Freisetzungsverzögerungsmittel ein Polymer ist.

5. Pharmazeutische Zusammensetzung nach Anspruch 4, wobei das Polymer eine Einfriertemperatur von weniger als dem Schmelzpunkt von Imatinibmesylat aufweist.

6. Pharmazeutische Zusammensetzung nach Anspruch 5, die ferner ein Plastifizierungsmittel umfasst.

7. Pharmazeutische Zusammensetzung nach Anspruch 1, wobei das Freisetzungsverzögerungsmittell ein nichtpolymeres Freisetzungsverzögerungsmittel ist.

8. Pharmazeutische Zusammensetzung nach Anspruch 7, wobei das nichtpolymere Freisetzungsverzögerungsmittel bei einer Temperatur schmilzt, die niedriger als der Schmelzpunkt von Imatinib oder dem verwendeten Imatinibsalz ist.

9. Pharmazeutische Zusammensetzung nach Anspruch 7, wobei das genannte nichtpolymere Freisetzungsverzögerungsmittel ein hydriertes Rizinusöl ist.

10. Pharmazeutische Zusammensetzung nach Anspruch 1, wobei die pharmazeutische Zusammensetzung mindestens ein Freisetzungsverzögerungsmittel umfasst, das aus der Gruppe ausgewählt ist, die aus wasserlöslichen, wasserunlöslichen und in Wasser quellbaren Cellulosepolymeren, Acrylpolymeren, Polysacchariden und Polyolen besteht.

11. Pharmazeutische Zusammensetzung nach Anspruch 1, wobei die pharmazeutische Zusammensetzung mindestens ein Freisetzungsverzögerungsmittel umfasst, das aus der Gruppe ausgewählt ist, die aus Hydroxypropylcellulose, Hydroxypropylmethylcellulose, Ethylcellulose und Methacrylatpolymeren besteht.

12. Pharmazeutische Zusammensetzung nach einem der Ansprüche 1 bis 11, die ferner ein Freisetzungsmodifizierungsmittel umfasst.

13. Verfahren zur Herstellung einer oralen, für eine verlängerte Freisetzung sorgenden pharmazeutischen Zusammensetzung, das die Stufe eines Schmelzgranulierens von Imatinib oder einem beliebigen der Salze hiervon mit einem Freisetzungsverzögerungsmittel und optional einem Freisetzungsmodifizierungsmittel in einem Extruder unter Erwärmen auf eine Temperatur unter die Schmelztemperatur von Imatinib oder einem Salz hiervon unter Bildung von Schmelzgranulat umfasst, wobei die Zusammensetzung mindestens 50 Gew.-% Imatinib, bezogen auf die Zusammensetzung, umfasst.

14. Verfahren nach Anspruch 13, wobei das Schmelzgranulat abermals in den Extruder eingeführt wird, um mit oder ohne Freisetzungsverzögerungsmittel oder Freisetzungsmodifizierungsmittel oder Plastifizierungsmittel bei einer Temperatur von weniger als dem Schmelzpunkt von Imatinib oder dem verwendeten Imatinibsalz zu granulieren.

15. Verfahren nach Anspruch 13, das ferner ein Kühlen des genannten Schmelzgranulats auf eine gewünschte Temperatur umfasst, die niedriger ist als die Schmelzgranulationsverfahrenstemperatur.

16. Verfahren nach Anspruch 13, das ferner ein Verpressen des Schmelzgranulats zu einer Tablette umfasst.

17. Verfahren nach Anspruch 16, wobei die getrennt unter Verwendung unterschiedlicher Freisetzungsverzögerungsmittel und/oder Freisetzungsmodifizierungsmittel und/oder Plastifizierungsmittel bei unterschiedlichen Zusammensetzungen hergestellten Schmelzgranulate vermischt und zu einer Tablette verpresst werden.

18. Verfahren nach Anspruch 13, wobei der Extruder ein Doppelschneckenextruder ist.

19. Verfahren nach Anspruch 14, wobei das Freisetzungsverzögerungsmittel ein Polymer ist.

20. Verfahren nach Anspruch 19, wobei das Polymer Hydroxypropylcellulose ist.

21. Pharmazeutische Zusammensetzung nach einem der Ansprüche 1 bis 12, wobei die Arzneimittelfreisetzung aus der pharmazeutischen Zusammensetzung nicht größer als 80 % nach einer Stunde und nicht kleiner als 80 % nach 10 Stunden bei Testen unter Verwendung einer USP 1-Körbchenapparatur bei 50 U/min in 900 ml 0,1 N Salzsäure bei 37 °C ist.

22. Pharmazeutische Zusammensetzung nach einem der Ansprüche 1 bis 12, wobei die Arzneimittelfreisetzung aus der pharmazeutischen Zusammensetzung nicht größer als 80 % nach zwei Stunden und nicht kleiner als 80 % nach 8 Stunden bei Testen unter Verwendung einer USP 1-Körbchenapparatur bei 50 U/min in 900 ml 0,1 N Salzsäure bei 37 °C ist.

23. Pharmazeutische Zusammensetzung nach einem der Ansprüche 1 bis 12, wobei die Zusammensetzung bei gesunden Menschen einen mittleren Plasmakonzentrationswert liefert, der 3,5 µg Imatinib/ml bei Verabreichung 2 Stunden nach einem leichten Frühstück nicht übersteigt.

## Revendications

1. Composition pharmaceutique orale à libération prolongée, comprenant des granulés de fusion d'imatinib ou de l'un quelconque de ses sels et un retardateur de libération, et qui comprend au moins 400 mg de mésylate d'imatinib.

2. Composition pharmaceutique selon la revendication 1, comprenant entre 50% et 99% en poids d'imatinib.

3. Composition pharmaceutique selon la revendication 2, ladite composition comprenant entre 62% et 99% en poids d'imatinib.

4. Composition pharmaceutique selon la revendication 1, dans laquelle ledit retardateur de libération est un polymère.

5. Composition pharmaceutique selon la revendication 4, dans laquelle ledit polymère a une température de transition vitreuse inférieure au point de fusion du mésylate d'imatinib.

6. Composition pharmaceutique selon la revendication 5, comprenant en outre un plastifiant.

7. Composition pharmaceutique selon la revendication 1, dans laquelle ledit retardateur de libération est un retardateur de libération non polymère.

8. Composition pharmaceutique selon la revendication 7, dans laquelle ledit retardateur de libération non polymère fond à une température inférieure au point de fusion de l'imatinib ou du sel d'imatinib employé.

9. Composition pharmaceutique selon la revendication 7, dans laquelle ledit retardateur de libération non polymère est l'huile de ricin hydrogénée.

10. Composition pharmaceutique selon la revendication 1, ladite composition pharmaceutique comprenant au moins un retardateur de libération choisi dans le groupe constitué par les polymères cellulosiques, les polymères acryliques, les polysaccharides et les polyols solubles dans l'eau, insolubles dans l'eau et gonflables à l'eau.

11. Composition pharmaceutique selon la revendication 1, ladite composition pharmaceutique comprenant au moins un retardateur de libération choisi dans le groupe constitué par l'hydroxypropylcellulose, l'hydroxypropylméthylcellulose, l'éthylcellulose et les polymères de méthacrylate.

12. Composition pharmaceutique selon l'une quelconque des revendications 1 à 11, comprenant en outre un modificateur de libération.

13. Procédé de fabrication d'une composition pharmaceutique orale à libération prolongée, comprenant l'étape consistant à granuler à l'état fondu l'imatinib ou l'un quelconque de ses sels avec un retardateur de libération et éventuellement, un modificateur de libération dans une extrudeuse tout en chauffant à une température inférieure à la température de fusion de l'imatinib ou de son sel, pour former des granulés de fusion, et dans lequel la composition comprend au moins 50% d'imatinib en poids de la composition.

14. Procédé selon la revendication 13, dans lequel les granulés de fusion sont introduits une fois de plus dans l'extrudeuse pour une granulation plus poussée avec ou sans retardateur de libération ou modificateur de libération ou plastifiant, à une température inférieure au point de fusion de l'imatinib ou du sel d'imatinib employé.

15. Procédé selon la revendication 13, comprenant en outre le refroidissement desdits granulés de fusion à une température souhaitée qui est inférieure à la température du procédé de granulation à l'état fondu.

16. Procédé selon la revendication 13, comprenant en outre la compression des granulés de fusion en forme de comprimé.

17. Procédé selon la revendication 16, dans lequel les granulés de fusion fabriqués séparément à l'aide de différents retardateurs de libération et/ou modificateurs de libération et/ou plastifiants à différentes compositions sont mélangés et compressés en forme de comprimé.

18. Procédé selon la revendication 13, dans lequel ladite extrudeuse est une extrudeuse à deux vis.

19. Procédé selon la revendication 14, dans lequel le retardateur de libération est un polymère.

20. Procédé selon la revendication 19, dans lequel ledit polymère est l'hydroxypropylcellulose.

21. Composition pharmaceutique selon l'une quelconque des revendications 1 à 12, où la libération du médicament de la composition pharmaceutique n'est pas supérieure à 80% au bout de 1 heure, et n'est pas inférieure à 80% au bout de 10 heures, lorsqu'on la teste en utilisant un appareil à panier USP I à 50 tours/min dans 900 mL d'acide chlorhydrique 0, 1N à 37°C.

22. Composition pharmaceutique selon l'une quelconque des revendications 1 à 12, où la libération du médicament de la composition pharmaceutique n'est pas supérieure à 80% au bout de 2 heures et n'est pas inférieure à 80% au bout de 8 heures, lorsqu'on la teste en utilisant un appareil à panier USP 1 à 50 tours/min dans 900 mL d'acide chlorhydrique 0, 1N à 37°C.

23. Composition pharmaceutique selon l'une quelconque des revendications 1 à 12, ladite composition procurant, chez des humains sains, une valeur de concentration plasmatique moyenne ne dépassant pas les 3,5 µg d'imatinib/mL, lorsqu'elle est dosée 2 heures après un petit-déjeuner léger.
